(19) Europäisches Patentamt
European Patent Office
Office européen des brevets

(11) EP 1 097 704 A1

(12) DEMANDE DE BREVET EUROPEEN

(43) Date de publication:
**09.05.2001 Bulletin 2001/19**

(51) Int Cl.[7]: **A61K 7/48**, A61K 7/42, A61K 7/06, A61K 9/107, A61K 7/00

(21) Numéro de dépôt: **00403056.5**

(22) Date de dépôt: **03.11.2000**

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **05.11.1999 FR 9913912**

(71) Demandeur: **L'OREAL**
**75008 Paris (FR)**

(72) Inventeurs:
- **Lorant, Raluca**
**94320 Thiais (FR)**
- **Bara, Isabelle**
**75013 Paris (FR)**
- **Josso, Martin**
**75005 Paris (FR)**
- **Vernaire, Sandrine**
**92310 Sevres (FR)**

(74) Mandataire: **Stalla-Bourdillon, Bernard**
**NONY & ASSOCIES**
**3, rue de Penthièvre**
**75008 Paris (FR)**

# (54) Emulsions transparentes ou translucides, leur procédé de préparation et leur utilisation en cosmétique

(57) L'invention a pour objet une émulsion cosmétique, transparente ou translucide, comprenant une phase aqueuse, une phase grasse et un agent tensio-actif, ladite phase grasse contenant un mélange miscible d'au moins une huile cosmétique et d'au moins un composé fluoré volatil, ce dernier étant présent en une proportion telle que l'indice de réfraction de la phase grasse soit égal à +/-0,05 celui de la phase aqueuse.

L'invention se rapporte également au procédé de préparation de l'émulsion.

Utilisation de l'émulsion dans les soins de la peau, le conditionnement des cheveux et la protection solaire et/ou le bronzage artificiel.

EP 1 097 704 A1

## Description

La présente invention a pour objet des émulsions transparentes ou translucides qui sont de préférence du type huile-dans-l'eau (H/E) ou eau-dans-l'huile (E/H) à base d'au moins un composé fluoré volatil, leur procédé de préparation, et leur utilisation en cosmétique.

La présente invention concerne tout particulièrement des émulsions cosmétiques destinées notamment aux soins de la peau, au conditionnement des cheveux, à la protection solaire et/ou au bronzage artificiel.

Les émulsions, on le sait, sont essentiellement constituées de deux phases non miscibles, l'une grasse et l'autre aqueuse, et d'un tensio-actif dont le rôle est de stabiliser la dispersion de l'une des phases, qui se trouve présente sous la forme de gouttelettes dispersées, au sein de la phase continue. Selon la proportion des deux phases au sein de l'émulsion, celle-ci est dite du type huile-dans-l'eau, lorsque la phase aqueuse est la phase continue, ou dite du type eau-dans-l'huile, lorsque la phase grasse est la phase continue.

Les émulsions sont d'un usage fréquent en cosmétique car elles offrent une grande flexibilité dans les formulations et dans les domaines d'applications cosmétiques. Elles peuvent en effet inclure une grande variété d'ingrédients actifs de nature très différente et se présenter sous diverses formes telles que des crèmes ou des gels plus ou moins fluides ou épais.

Toutefois, ces émulsions présentent généralement une apparence blanchâtre, et sont de ce fait peu attractives aux yeux du consommateur. Cet aspect résulte essentiellement des phénomènes de déviation par réfraction et réflexion des rayons lumineux à l'interface des deux phases. Cette opacité est particulièrement observée dans le cas des produits solaires, certains filtres solaires ayant des indices de réfraction élevés, ce qui accentue les phénomènes de déviation.

Divers procédés ont été envisagés en vue de minimiser la déviation des rayons lumineux et donc d'augmenter la transparence des compositions.

Parmi ceux-ci, on peut citer les procédés qui consistent à réduire le diamètre des gouttelettes de la phase dispersée de l'émulsion, soit par inversion de phase, ce qui s'avère toutefois fastidieux, soit par addition de proportions élevées d'agents tensio-actifs et passage dans un appareil à haute pression. Les microémulsions ainsi obtenues présentent toutefois, dans ce dernier cas, l'inconvénient de provoquer certaines réactions cutanées d'irritation et d'inconfort, ce qui les rend cosmétiquement peu acceptables, du fait essentiellement de l'excès d'agent tensio-actif.

Il a également été proposé un procédé consistant à augmenter l'indice de réfraction de la phase aqueuse par addition de glycol ou de glycérol, ceci de façon à le rapprocher de celui de la phase grasse. Ce procédé nécessite néanmoins l'addition d'une proportion souvent élevée de glycol ou de glycérol et les émulsions ainsi obtenues présentent de réels désagréments tels qu'un toucher cosmétique collant, ainsi que des problèmes d'inconfort lors de l'application.

Par ailleurs, l'addition de glycol ou de glycérol peut ne pas être suffisante pour obtenir des compositions ayant une bonne transparence du fait de l'indice de réfraction généralement élevé de certains filtres solaires.

On sait par ailleurs préparer des compositions transparentes, solaires ou autobronzantes, sous forme de gels aqueux ou hydroalcooliques, toutefois on ne peut utiliser dans les gels aqueux des filtres solaires liposolubles et ces gels ne sont que très faiblement résistants à l'eau.

Les gels hydroalcooliques présentent eux l'inconvénient de contenir des proportions élevées en alcool, notamment en éthanol, ce qui soulève des problèmes techniques lors de leur formulation.

On a maintenant constaté de façon surprenante et inattendue, qu'il était possible d'obtenir d'excellentes émulsions transparentes ou translucides en abaissant l'indice de réfraction de la phase grasse afin qu'il soit sensiblement égal à celui de la phase aqueuse, et ceci en utilisant un composé fluoré volatil miscible, d'indice de réfraction inférieur ou égal à 1,3.

La présente invention a donc pour objet une émulsion transparente ou translucide, comprenant une phase aqueuse, une phase grasse et un agent tensioactif, caractérisée par le fait que la phase grasse contient un mélange miscible d'au moins une huile cosmétique et d'au moins un composé fluoré volatil, ce dernier étant présent en une proportion telle que l'indice de réfraction de la phase grasse soit égal à +/-0,05 celui de la phase aqueuse, de préférence égal à +/- 0,03 et de préférence encore égal à +/- 0,01.

Par "émulsion transparente ou translucide", on entend selon l'invention, une émulsion dont la matrice laisse passer la lumière sans provoquer de déviation par réfraction ou réflexion, ou en ne provoquant que de faibles déviations des rayons lumineux à l'interface des deux phases. Si la transparence d'une émulsion peut être facilement évaluée à l'oeil nu, elle est généralement mesurée à l'aide d'un Turbidimètre. Le Turbidimètre portatif Modèle 2100P® de la Société HACH peut par exemple être utilisé pour les mesures des gammes de transparence des émulsions selon la présente invention. Celles-ci sont dites transparentes lorsque la valeur mesurée est comprise entre 0 et 250 NTU, alors qu'elles sont dites translucides pour une valeur allant de 250 à 1000 NTU.

Par l'expression "miscible", on doit entendre selon l'invention que le mélange se présente sous forme homogène, c'est-à-dire qu'il ne se produit pas de déphasage entre les constituants après leur mise en contact.

Par l'expression "huile cosmétique", on doit entendre selon l'invention tous corps gras liquides à 25 °C, cosmétiquement acceptables.

Le composé fluoré volatil de la phase grasse, utilisé en mélange avec au moins une huile cosmétique, est de préférence choisi parmi les composés suivants :

1°) Les perfluorocycloalkyles répondant à la formule (I) suivante :

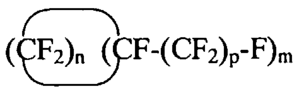 (I)

dans laquelle :

n est 2, 3, 4 ou 5
m est 1 ou 2, et
p est 1, 2 ou 3

sous réserve que lorsque n = 2, m est 2 et que lorsque n = 3, 4 ou 5 et m=2, les groupes perfluoroalkyles ne sont pas nécessairement en alpha, l'un par rapport à l'autre ;

2°) Les perfluoroalcanes répondant à la formule (II) suivante :

$$CF_3\text{-}(CF_2)_m\text{-}CF_2X \qquad (II)$$

dans laquelle :

m est un entier compris entre 2 à 8, et
X représente Br ou F ;

3°) Les fluoroalkyles ou hétérofluoroalkyles répondant à la formule (III) suivante :

$$CH_3\text{-}(CH_2)_n\text{-}[Z]_t\text{-}X\text{-}CF_3 \qquad (III)$$

dans laquelle :

X est un radical perfluoroalkyle, linéaire ou ramifié, ayant de 2 à 5 atomes de carbone, et
Z est O, S ou NR, R étant un hydrogène, un radical $-(CH_2)_n-CH_3$, ou $-(CF_2)_m-CF_3$, m étant 2, 3, 4 ou 5, n est 0, 1, 2 ou 3 et t est 0 ou 1, et

4°) les dérivés de perfluoromorpholine répondant à la formule (IV) suivante :

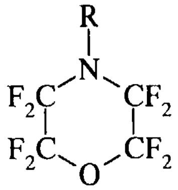 (IV)

dans laquelle R est un radical perfluoroalkyle en $C_1$-$C_4$.

Parmi les perfluorocycloalkyles de formule (I), on peut notamment citer le perfluorométhylcyclopentane et le perfluoro-1,3 diméthylcyclohexane, qui sont vendus sous les dénominations de "FLUTEC PC1®" et "FLUTEC PC3®" par la Société BNFL FLUOROCHEMICALS Ltd., et le perfluoro-1,2-diméthylcyclobutane.

Parmi les perfluoroalcanes de formule (II), on peut citer, entre autres, le dodécafluoropentane et le tétradécafluorohexane, vendus sous les dénominations de "PF 5050®" et "PF 5060®" par la Société 3M ou encore le bromoperfluorooctyle vendu sous la dénomination de "FORALKYL®" par la Société ATOCHEM.

Parmi les composés fluorés de formule (III), on peut citer par exemple, le méthoxynonafluorobutane et l'éthoxynonafluorobutane vendus respectivement sous les dénominations de "HFE-7100®" et "HFE-7200®" par la Société 3M.

Enfin, parmi les dérivés de perfluoromorpholine de formule (IV), on peut citer par exemple la 4-trifluorométhyl perfluoromorpholine vendue sous la dénomination "PF 5052®" par la Société 3M.

Ces composés fluorés volatils tels qu'ils viennent d'être définis ci-dessus ont tous un indice de réfraction ($n_D^{20}$) inférieur à 1,43, de préférence inférieur à 1,40 et tout particulièrement inférieur à 1,36 ; ils ont de préférence une pression de vapeur saturante, à 25°C, au moins égale à 50 Pa ; de plus ils ont un point d'ébullition généralement compris entre 25 et 65°C et une densité élevée généralement supérieure à 1, de préférence supérieure à 1,2.

Le composé fluoré volatil est présent dans la phase grasse de telle sorte que l'indice de réfraction de ladite phase soit de préférence inférieure à 1,36, sa proportion variant bien entendu, en fonction de son indice de réfraction propre et de celui de l'huile ou du mélange d'huiles cosmétiques. Toutefois, sa proportion est généralement d'au moins 5 % et de préférence comprise entre environ 8 et 95 % en poids par rapport au poids total de ladite phase, le reste étant constitué par l'huile ou le mélange d'huiles cosmétiques.

Selon l'invention, on préfère utiliser des composés fluorés volatils dont les paramètres de solubilité sont tels qu'ils permettent de multiples associations homogènes avec d'autres huiles cosmétiques d'indice de réfraction plus élevé notamment.

Ainsi, on peut choisir de préférence des composés fluorés volatils dont les paramètres de solubilité, selon l'espace de HANSEN, répondent aux critères suivants :

$\delta d \geq 13$ ; $\delta p \geq 7$ et $\delta h \geq 3$, mesurés à une concentration supérieure à 10 % dans un mélange de solvant.

L'huile cosmétique miscible avec le composé fluoré volatil se présente sous forme liquide à 25 °C et peut être choisie parmi des huiles volatiles ou non volatiles, en particulier des huiles hydrocarbonées d'origine animale telles que le perhydrosqualène, des huiles hydrocarbonées végétales telles que les triglycérides liquides d'acides gras en $C_4$-$C_{10}$, des hydrocarbures, linéaires ou ramifiés, d'origine minérale ou synthétique, des esters ou éthers de synthèse, des alcools gras en $C_{12}$-$C_{26}$, des huiles fluorées éventuellement partiellement hydrocarbonées et/ou siliconées, lesdites huiles fluorées étant de structure et de propriétés différentes de celles du composé fluoré volatil.

Parmi les huiles cosmétiques particulièrement préférées, on peut mentionner les polysiloxanes en particulier les PDMS et les huiles siliconées volatiles, notamment les silicones volatiles cycliques ou linéaires telles que le cyclotétrasiloxane, le cyclopentasiloxane et le cyclohexasiloxane et les huiles volatiles hydrocarbonées telles que les isoparaffines en $C_{11}$-$C_{13}$ et notamment l'isododécane, ainsi que les mélanges desdites huiles.

Les émulsions selon l'invention peuvent être du type huile-dans-l'eau ou eau-dans-huile.

Lorsque les émulsions sont du type huile-dans-l'eau, la proportion de la phase aqueuse est généralement comprise entre 5 et 95 %, de préférence entre 50 et 95 %, et celle de la phase grasse est comprise entre 0,2 et 94 %, de préférence entre 0,5 et 50 %, l'agent tensio-actif étant présent en une proportion de 0,1 à 10 % en poids.

Par contre, lorsque les émulsions sont du type eau-dans-l'huile, la proportion de la phase grasse est généralement comprise entre 10 et 90 % et la phase eau entre 10 et 90 %, l'agent tensio-actif étant présent en une proportion de 0,1 à 10 % en poids.

L'agent tensio-actif de ces émulsions est généralement choisi parmi ceux du type hydrocarboné, siliconé et/ou fluoré.

Lorsque l'on souhaite obtenir une émulsion du type huile-dans-l'eau, on utilise de préférence un agent tensio-actif anionique ou amphotère comprenant au moins un groupement phosphate ou sulfate tel que ceux décrits dans la demande FR-2 745 715, dont le contenu est incorporé par référence dans la présente description. Parmi les agents tensio-actifs de ce type, on peut citer ceux commercialisés sous les dénominations "PECOSIL PS-100®", PECOSIL PS-200®", et "PECOSIL WDS-100®" par la Société PHOENIX CHEMICAL. Parmi les autres tensio-actifs pouvant conduire à des émulsions H/E, on peut également citer les tensio-actifs fluorés et notamment la polyfluorohexylbétaïne vendue sous la dénomination de "FORAFAC I157®" par la société ATOCHEM.

Par contre, lorsque l'on souhaite obtenir une émulsion du type eau-dans-l'huile on utilise de préférence un agent tensio-actif siliconé, en particulier ceux appartenant à la famille des alkyl- ou alcoxy-diméthicones copolyols ou encore des diméthicones copolyols tels que ceux décrits dans la demande FR-2 701 845. Parmi ces tensio-actifs, on peut mentionner ceux vendus sous les dénominations de "ABIL WE09®", "ABIL WS08®", "ABIL EM90®" ou "ABIL EM97®" par la Société GOLDSCHMIDT, de "Q2-5200®", "DC-3225C®" ou "DC-5225®" par la Société Dow CORNING et de "218-1138®", de "SF1228®" ou de "SF1328®" par la Société GENERAL ELECTRIC.

Les émulsions selon l'invention sont particulièrement utiles en cosmétique du fait de leur apparence agréable et de leur texture polysensorielle alliant fraîcheur et douceur.

Les émulsions selon l'invention peuvent se présenter sous forme de produits de soins pour la peau, de produits solaires et/ou auto-bronzants ou encore de produits pour le conditionnement des cheveux.

On a en effet constaté que l'utilisation de composés fluorés volatils permettait non seulement d'obtenir des émulsions ayant une bonne transparence, mais conférait également des propriétés cosmétiques d'agrément et de confort ainsi qu'un effet immédiat de fraîcheur dès l'application.

Par ailleurs, l'utilisation d'au moins un composé fluoré volatil permet d'obtenir un film non collant, non graisseux, particulièrement léger et cosmétique, pouvant convenir à des applications dans des pays chauds et humides.

Lorsque les émulsions sont destinées aux soins de la peau, celles-ci peuvent contenir un ou plusieurs actifs cosmétiques, solubles dans l'une des phases, tels que par exemple des agents hydratants, des agents humectants, des agents émollients, des agents régénérants, des agents amincissants, des agents décongestionnants, des agents anti-inflammatoires, des agents éclaircissants, des agents détoxifiants, des agents cicatrisants, ou des agents adoucissants.

Lorsque les émulsions sont sous forme de produits de protection solaire et/ou auto-bronzants, on peut y incorporer une grande variété de filtres solaires lipophiles ou hydrophiles, notamment des filtres à indice de réfraction élevé, sans que l'aspect de l'émulsion n'en soit affecté. De plus, ces émulsions présentent une très bonne résistance à l'eau et à la transpiration, et ne laissent aucune sensation grasse résiduelle.

Selon ce mode de réalisation particulier, les émulsions contiennent un ou plusieurs filtres solaires organiques parmi lesquels on peut notamment citer : les dérivés cinnamiques ; les dérivés de dibenzoylméthane ; les dérivés salicyliques ; les dérivés du camphre ; les dérivés de triazinc tels que ceux décrits dans US 4.367.390, EP 863.145, EP 517.104, EP 570.838, EP 796.851, EP 775.698, EP 878.469 et EP 933.376, les dérivés de la benzophénone ; les dérivés de β,β'diphénylacrylate ; les dérivés de benzimidazole ; les dérivés bis-benzoazolyle tels que ceux décrits dans EP-A-669.323 et US 2.463.264 ; les dérivés de bis-hydroxyphénolbenzotriazole tels que ceux décrits dans US 5.237.071, US 5.166.355, GB-A-2.303.549, DE 197.26.184 et EP-A-893.119 ; les dérivés de l'acide p-aminobenzoïque ; les polymères hydrocarbonés filtres et les silicones filtres tels que ceux décrits notamment dans WO-93/04665.

Comme exemples de filtres solaires actifs dans l'UV-A et/ou l'UV-B, on peut citer :

- l'acide p-aminobenzoïque,
- le p-aminobenzoate oxyéthyléné (25mol),
- le p-diméthylaminobenzoate d'éthyl-2 hexyle,
- le p-aminobenzoate d'éthyle N-oxypropyléné,
- le p-aminobenzoate de glycérol,
- le salicylate d'homomenthyle,
- le salicylate d'éthyl-2 hexyle,
- le salicylate de triéthanolamine,
- le salicylate d'isopropyl-4 benzyle,
- le tert-butyl-4 méthoxy-4' dibenzoylméthane,
- l'isopropyl-4 dibenzoyl méthane,
- le méthoxy-4 cinnamate d'éthyl-2 hexyle,
- le diisopropyl cinnarnate de méthyle,
- le méthoxy-4 cinnamate d'isoamyle,
- le méthoxy-4 cinnamate de diéthanolamine,
- l'anthranilate de menthyle,
- le diphényl-3, 3 cyano-2 acrylate d'éthyl-2 hexyle,
- le diphényl-3, 3 cyano-2 acrylate d'éthyle,
- l'acide phényl-2 benzimidazole sulfonique-5 et ses sels,
- le 3 -(4'-triméthylammonium)-benzylidène-bornan-2-one-méthylsulfate,
- l'hydroxy-2 méthoxy-4 benzophénone,
- l'hydroxy-2 méthoxy-4 benzophénone sulfonate-5,
- la dihydroxy-2,4 benzophénone
- la tétrahydroxy-2,2',4,4' benzophénone,
- la dihydroxy-2,2' diméthoxy-4,4' benzophénone,
- l'hydroxy-2(n-octyloxy)-4 benzophénone,
- l'hydroxy-2 méthoxy-4-méthy-4' benzophénone,
- l'acide a-(2-oxobom-3-ylidène)-tolyl-4-sulfonique et ses sels solubles,
- la 3-(4'-sulfo)benzylidèn-bornan-2-one et ses sels solubles,
- le (méthyl-4' benzylidène)-3 d,1-camphre,
- le benzylidène-3-d,l-camphre,
- l'acide benzène di-1,4(méthylidène-3 camphosulfonique-10) et ses sels solubles,
- l'acide urocanique,
- la tris-2,4,6-[ p-(-éthyl-2' hexyl-oxycarbonyl-1') anilino] triazine-1,3,5,
- la [p-(tert-butylamido) anilino]-2-bis-[(p-(éthyl-2' hexyl-oxycarbonyl-1') anilino]-4,6- triazine-1,3,5,
- la 2,4-bis {[éthyl-2 hexyloxy-4)] hydroxyl-2 phényl} (méthoxy-4 phényl)-6 triazine-1,3,5-,
- le polymère de N-(2 et 4)-[(2-oxoborn-3-ylidèn)méthyl] benzyl]-acrylamide,
- l'acide 1,4-bis-benzimidazolyl-phénylen-3,3', 5,5'-tétrasulfonique et ses sels solubles,
- le 2,-2'-méthylène-bis- [6- (2H - benzotriazol-2-yl) -4- (1,1,3,3-tétraméthyl butyl) phénol],
- le (2,2'-méthylène-bis-[6-(2H-benzotriazol-2-yl)-4-(methyl)phénol],
- les polyorganosiloxanes à fonction benzalmalonate,
- les polyorganosiloxanes à fonction benzotriazole tel que le "Drometrizole Trisiloxane®".

Parmi ces différents filtres, on préfère utiliser selon l'invention en tant que filtres solaires lipophiles le p-méthoxycinnamate d'éthyl-2 hexyle vendu sous la dénomination de "PARSOL MCX®" par la société GIVAUDAN-ROURE, le diphényl-3,3 cyano-2 acrylate d'éthyl-2 hexyle vendu sous la dénomination de "UVINUL N539®" par la société BASF ou le tert-butyl-4 méthoxy-4' dibenzoyl méthane vendu sous la dénomination de "PARSOL 1789®" par la société GIVAUDAN-ROURE et en tant que filtres solaires hydrophiles l'acide téréphthalylidène dicamphre sulfonique vendu sous la dénomination de "MEXORYL SX®" par la société CHIMEX ou l'acide de phényl-2 benzimidazole sulfonique-5 vendu sous la dénomination de "EUSOLEX 232®" par la société RONA/MERCK.

Les émulsions peuvent également contenir un ou plusieurs agents auto-bronzants tels que par exemple la dihydroxyacétone (DHA).

Les émulsions de soins ou de protection solaire et/ou auto-bronzantes selon l'invention peuvent également contenir tout autre additif cosmétique classique.

Parmi ceux-ci, on peut citer par exemple les conservateurs, les antioxydants, les polymères filmogènes, les colorants solubles, et les vitamines. L'objet de l'invention étant l'obtention d'émulsions transparentes ou translucides, il va de soi que celles-ci sont quasiment exemptes de tous produits insolubles et en particulier de pigment, de charge ou de poudre.

Selon la consistance que l'on souhaite obtenir, les émulsions peuvent éventuellement contenir au moins un agent épaississant en une proportion d'environ 0,05 à 10 %, mais de préférence comprise entre 0,1 et 5 % en poids.

L'agent épaississant peut être notamment choisi parmi :

- les biopolymères polysaccharidiques comme la gomme de xanthane, la gomme de caroube, la gomme de guar, les alginates, les celluloses modifiées telles que l'hydroxyéthylcellulose, la méthylcellulose, l'hydroxypropylcellulose, et la carboxyméthylcellulose ;
- les polymères synthétiques comme les acides polyacryliques tels que les polymères poly(méth) acrylates de glycéryle vendus sous les dénominations "HISPAGEL®", ou "LUBRAGEL®" par la Société HISPANO QUIMICA ou la Société GUARDIAN, la polyvinylpyrrolidone, l'alcool polyvinylique, les polymères réticulés d'acrylamide et d'acrylate d'ammonium vendus sous les dénominations "PAS 5161®" ou "BOZEPOL®" par la Société HOECHST, les polymères réticulés d'acrylamide et de chlorure de méthacryloyloxyéthyltriméthylammonium vendus sous la dénomination "SALCARE SC 92®" par la Société ALLIED COLLOIDS, et le silicate de magnésium d'aluminium.

On utilise de préférence, en tant qu'agent épaississant, les polymères réticulés d'acrylamide et d'acide 2-acrylamido 2-méthylpropane sulfonique, également désignés polymères d'AMPS, partiellement ou totalement neutralisés et vendus sous la dénomination de "SEPIGEL 305®" par la Société SEPPIC. Ceux-ci sont notamment décrits dans la demande EP-503 853, dont le contenu est incorporé par référence dans la présente description.

La présente invention a également pour objet le procédé de préparation des émulsions cosmétiques, transparentes ou translucides, telles que définies ci-dessus.

Ce procédé consiste à ajuster l'indice de réfraction de la phase grasse, contenant au moins une huile cosmétique, à l'aide d'au moins un composé fluoré volatil tel que défini précédemment, ce dernier étant présent dans ladite phase grasse en une concentration telle que l'indice de réfraction de cette dernière soit égal à +/-0,05 celui de la phase aqueuse de l'émulsion.

Plus précisément, le procédé de préparation des émulsions, transparentes ou translucides, selon l'invention, comprend les étapes consistant :

(a) à préparer la phase grasse à base d'au moins une huile cosmétique miscible avec le composé fluoré volatil,
(b) à préparer séparément la phase aqueuse,
(c) à ajouter à l'une desdites phases au moins un agent tensio-actif,
(d) à mesurer à l'aide d'un réfractomètre l'indice de réfraction desdites phases grasse et aqueuse,
(e) à ajuster l'indice de réfraction de la phase grasse de sorte qu'il soit égal à +/-0,05 celui de la phase aqueuse, par addition du composé fluoré volatil,
(f) à émulsionner, sous forte agitation, et dans l'ordre voulu, ladite phase aqueuse (b) et ladite phase grasse modifiée (e), à la température ambiante ou sous refroidissement, et
(g) à introduire éventuellement lors de la formation de l'émulsion tout ingrédient cosmétique conventionnel.

On va maintenant donner à titre d'illustration plusieurs exemples d'émulsions, transparentes ou translucides, sous forme de produits de soins de la peau, de produits solaires et/ou auto-bronzants ou encore de produits pour le conditionnement des cheveux.

## EXEMPLES

### EXEMPLE 1 : Crème de soins transparente

Cette crème sous forme d'une émulsion H/E a été obtenue après préparation des phases aqueuse et grasse suivantes :

A - Préparation de la phase aqueuse :

La phase aqueuse a été obtenue par mélange des ingrédients suivants :

- Diméthicone copolyol phosphate ("PECOSIL PS100®") 2,40 %
- Soude 0,06 %
- Conservateurs 0,20 %
- Eau 75,30 %

Indice de réfraction de la phase aqueuse $n_D^{20}$ = 1,345.

B - Préparation de la phase grasse :

La phase grasse a été obtenue à partir de cyclohexasiloxane d'indice de réfraction 1,405. Celui-ci a été abaissé par addition de nonafluorométhoxybutane pour conduire au mélange suivant :

- Cyclohexasiloxane 8,91 %
- Méthoxynonafluorobutane ("HFE-7100®") 10,88 %

L'indice de réfraction de la phase grasse, après mélange, était de : $n_D^{20}$ = 1,345, c'est-à-dire identique à celui de la phase aqueuse.

On a alors procédé sous forte agitation et à température ambiante à la formation de l'émulsion en introduisant la phase grasse à la phase aqueuse et on a simultanément procédé à l'addition des ingrédients suivants :

- Gomme de xanthane 0,2 %
- Polymère d'AMPS ("SEPIGEL 305®") 2,0 %

Après avoir poursuivi l'agitation quelques minutes, on a obtenu une émulsion ayant une très bonne transparence et d'excellentes propriétés cosmétiques.

### EXEMPLE 2 : Emulsion H/E transparente

Selon le même mode opératoire que celui décrit à l'Exemple 1, on a préparé une émulsion H/E transparente à l'aide des phases aqueuse et grasse suivantes :

A - Phase aqueuse :

- Polyfluorohexylbétaïne ("FORAFAC 1157®") 7 %
- Conservateurs 0,2 %
- Eau 68,8 %

Indice de réfraction de la phase aqueuse $n_D^{20}$ = 1,334.

B - Phase grasse :

- Méthoxynonafluorobutane ("HFE-7100®") 12 %
- Huile de paraffine 12 %

Indice de réfraction de la phase grasse $n_D^{20}$ = 1,368.

Après homogénéisation des deux phases sous forte agitation, on a obtenu une émulsion H/E d'excellente transparence.

**EXEMPLE 3 : Emulsion E/H anti-solaire transparente**

On a préparé une émulsion E/H anti-solaire transparente à l'aide des phases aqueuse et grasse suivantes :

A - Phase aqueuse :

- Glycol 5 %
- Propylène glycol 31,9 %
- Dipropylène glycol 5 %
- Filtre UV hydrosoluble ("EUSOLEX 232®") 3 %
- Soude 0,4 %
- NaCl 2 %
- Eau 20,7 %

Indice de réfraction de la phase aqueuse $n_D^{20}$ = 1,428.

B - Phase grasse :

- Méthoxynonafluorobutane ("HFE-7100®") 6 %
- Isododécane 6 %
- Huile de silicone (mélange PDMS $\alpha$-$\omega$ dihydroxylé/PDMS) 3 %
- Diméthicone copolyol ("DC-3225C®") 10 %
- Filtre UV liposoluble ("PARSOL MCX®") 7 %

Indice de réfraction de la phase grasse $n_D^{20}$ = 1,421.

On a alors procédé, sous forte agitation et à température ambiante, à la formation de l'émulsion en introduisant la phase aqueuse à la phase grasse.

Après homogénéisation des deux phases, on obtient une émulsion E/H anti-solaire de très bonne transparence.

**EXEMPLE 4 : Emulsion E/H translucide**

On a préparé une émulsion E/H translucide en procédant à l'introduction, sous forte agitation, de 67,8 g d'eau dans une phase grasse homogène constituée par :

- Cyclométhicone (cyclopentasiloxanc) 15 g
- Ethoxynonafluorobutane ("HFE-7200®") 15 g
- Cétyldiméthicone copolyol ("ABIL EM 90®") 2,2 g

Indice de réfraction de la phase grasse : $n_D^{20}$ = 1,351.

Après homogénéisation des deux phases, on obtient une émulsion de bonne translucidité.

## Revendications

1. Emulsion cosmétique, transparente ou translucide, comprenant une phase aqueuse, une phase grasse et un agent tensio-actif, caractérisée par le fait que la phase grasse contient un mélange miscible d'au moins une huile cosmétique et d'au moins un composé fluoré volatil, ce dernier étant présent en une proportion telle que l'indice de réfraction de la phase grasse soit égal à +/-0,05 celui de la phase aqueuse.

2. Emulsion selon la revendication 1, caractérisée par le fait que le composé fluoré volatil est un composé perfluorocycloalkyle répondant à la formule (I) suivante :

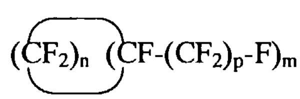 (I)

dans laquelle :

n est 2, 3, 4 ou 5,
m est 1 ou 2, et
p est1, 2 ou 3,

sous réserve que lorsque n = 2, m est 2 et que lorsque n = 3, 4 ou 5 et m=2, les groupes perfluoroalkyles ne sont pas nécessairement en alpha, l'un par rapport à l'autre.

3. Emulsion selon la revendication 1, caractérisée par le fait que le composé fluoré volatil est un perfluoroalcane de formule (II) suivante :

$$CF_3\text{-}(CF_2)_m\text{-}CF_2X \qquad (II)$$

dans laquelle :

m est un entier compris entre 2 à 8, et
X représente Br ou F.

**4.** Emulsion selon la revendication 1, caractérisée par le fait que le composé fluoré volatil est un fluoroalkyle ou un hétérofluoroalkyle répondant à la formule (III) suivante :

$$CH_3\text{-}(CH_2)_n\text{- }[Z]_t\text{ - }X\text{-}CF_3 \qquad (III)$$

dans laquelle :

X est un radical perfluoroalkyle linéaire ou ramifié, ayant 2 à 5 atomes de carbone, et
Z est O, S ou NR, R étant un hydrogène, un radical $-(CH_2)_n-CH_3$ ; ou $-(CF_2)_m-CF_3$, m étant 2, 3, 4 ou 5, n est 0, 1, 2 ou 3 et t est 0 ou 1.

**5.** Emulsion selon la revendication 1, caractérisée par le fait que le composé fluoré volatil est un dérivé perfluoromorpholine répondant à la formule (IV) suivante :

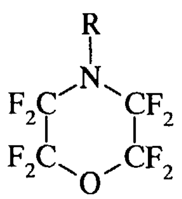

(IV)

dans laquelle R est un radical perfluoroalkyle en $C_1$-$C_4$.

**6.** Emulsion selon l'une quelconque des revendications précédentes, caractérisée par le fait que le composé fluoré volatil est choisi dans le groupe constitué par le perfluorométhylcyclopentane et le perfluoro-1,3 diméthylcyclohexane, le perfluoro-1,2-diméthylcyclobutane, le dodécafluoropentane, le tétradécafluorohexane, le bromoperfluorooctyle, le méthoxynonafluorobutane, l'héthoxynonafluorobutane et la 4-trifluorométhyl perfluoromorpholine.

**7.** Emulsion selon l'une quelconque des revendications précédentes, caractérisée par le fait que le composé fluoré volatil est présent dans ladite phase grasse en une proportion d'au moins 5 % en poids par rapport au poids total de ladite phase.

**8.** Emulsion selon l'une quelconque des revendications précédentes, caractérisée en ce que ladite huile cosmétique miscible est choisie parmi le cyclotétrasiloxane cyclopentasiloxane, le cyclohexasiloxane, l'isododécane et les mélanges desdites huiles.

**9.** Emulsion selon l'une quelconque des revendications précédentes, caractérisée par le fait qu'elle contient en outre au moins un principe actif cosmétique.

**10.** Emulsion selon l'une quelconque des revendications précédentes, caractérisée par le fait qu'elle contient également au moins un filtre solaire et/ou un agent auto-bronzant.

**11.** Emulsion selon l'une quelconque des revendications précédentes, caractérisée par le fait qu'elle contient également au moins un ingrédient cosmétique conventionnel.

**12.** Emulsion selon l'une quelconque des revendications précédentes, caractérisée par le fait qu'elle contient également un agent épaississant.

**13.** Emulsion selon l'une quelconque des revendications précédentes, caractérisée en ce qu'elle se présente sous la forme d'un produit de soins pour la peau, d'un produit de protection solaire et/ou d'un produit auto-bronzant ou d'un produit de conditionnement pour les cheveux.

**14.** Procédé de préparation d'une émulsion cosmétique, transparente ou translucide, selon l'une quelconque des revendications 1 à 13, caractérisé par le fait que l'on ajuste l'indice de réfraction de la dite phase grasse, contenant au moins une huile cosmétique à l'aide dudit composé fluoré volatil, ce dernier étant présent en une proportion telle que l'indice de réfraction de ladite phase grasse soit égal à ± 0,05 celui de ladite phase aqueuse.

**15.** Procédé selon la revendication 14, caractérisé par le fait qu'il consiste en les étapes suivantes :

(a) à préparer ladite phase grasse à base d'au moins une huile cosmétique miscible avec le composé fluoré volatil,
(b) à préparer séparément ladite phase aqueuse,
(c) à ajouter à l'une desdites phases ledit agent tensio-actif,
(d) à mesurer l'indice de réfraction desdites phases grasse et aqueuse,
(e) à ajuster l'indice de réfraction de ladite phase grasse de sorte qu'il soit égal à +/-0,05 celui de la phase aqueuse, par addition dudit composé fluoré volatil,
(f) à émulsionner, sous forte agitation, dans l'ordre voulu, ladite phase aqueuse (b) et ladite phase grasse modifiée (e), à la température

ambiante ou sous refroidissement, et
(g) à introduire éventuellement lors de la formation de l'émulsion au moins un ingrédient cosmétique conventionnel.

**16.** Emulsion cosmétique transparente ou translucide, du type huile-dans-l'eau ou eau-dans-l'huile susceptible d'être obtenue par le procédé selon les revendications 14 et 15.

Office européen des brevets

# RAPPORT DE RECHERCHE EUROPEENNE

Numéro de la demande
EP 00 40 3056

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | EP 0 639 371 A (OREAL)<br>22 février 1995 (1995-02-22)<br>* revendications; exemples *<br>--- | 1-13,16 | A61K7/00<br>A61K7/48<br>A61K7/06 |
| A | FR 2 773 064 A (OREAL)<br>2 juillet 1999 (1999-07-02)<br>* revendications 1-12 *<br>--- | 1-13,16 | |
| A | EP 0 609 131 A (OREAL)<br>3 août 1994 (1994-08-03)<br>* revendications 1,3,10 *<br>----- | 1 | |

DOMAINES TECHNIQUES RECHERCHES (Int.Cl.7)
A61K

Le présent rapport a été établi pour toutes les revendications

| Lieu de la recherche | Date d'achèvement de la recherche | Examinateur |
|---|---|---|
| BERLIN | 12 janvier 2001 | Beyss, E |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

**ANNEXE AU RAPPORT DE RECHERCHE EUROPEENNE**
**RELATIF A LA DEMANDE DE BREVET EUROPEEN NO.** EP 00 40 3056

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

12-01-2001

| Document brevet cité au rapport de recherche | Date de publication | Membre(s) de la famille de brevet(s) | Date de publication |
|---|---|---|---|
| EP 0639371 A | 22-02-1995 | FR 2709060 A | 24-02-1995 |
| | | AT 167394 T | 15-07-1998 |
| | | CA 2130368 A | 21-02-1995 |
| | | DE 69411098 D | 23-07-1998 |
| | | DE 69411098 T | 11-02-1999 |
| | | ES 2119980 T | 16-10-1998 |
| | | JP 2766783 B | 18-06-1998 |
| | | JP 7173025 A | 11-07-1995 |
| | | US 6132736 A | 17-10-2000 |
| FR 2773064 A | 02-07-1999 | FR 2773065 A | 02-07-1999 |
| | | BR 9805691 A | 16-05-2000 |
| | | EP 0930059 A | 21-07-1999 |
| | | JP 11263710 A | 28-09-1999 |
| | | BR 9805721 A | 11-04-2000 |
| | | EP 0930058 A | 21-07-1999 |
| | | FR 2773066 A | 02-07-1999 |
| | | JP 11263709 A | 28-09-1999 |
| EP 0609131 A | 03-08-1994 | FR 2700691 A | 29-07-1994 |
| | | AT 161170 T | 15-01-1998 |
| | | DE 69407316 D | 29-01-1998 |
| | | DE 69407316 T | 09-04-1998 |
| | | ES 2110703 T | 16-02-1998 |
| | | JP 6234615 A | 23-08-1994 |
| | | US 5741499 A | 21-04-1998 |

EPO FORM P0460

Pour tout renseignement concernant cette annexe : voir Journal Officiel de l'Office européen des brevets, No.12/82